# EUROPEAN PATENT SPECIFICATION

(11) **EP 1 962 949 B1**
(45) Date of publication and mention of the grant of the patent: **25.02.2015**
(21) Application number: 06847759.5
(22) Date of filing: 19.12.2006
(51) Int. Cl.: A61N 1/05, A61N 1/36, A61F 2/07, A61F 2/82, A61F 2/89, A61F 2/90

(54) **APPARATUS FOR MODULATING THE BAROREFLEX SYSTEM**
VORRICHTUNG ZUR MODULATION DES BAROREFLEXSYSTEMS
APPAREIL DE MODULATION DU SYSTEME BAROREFLEXE

(30) Priority: 20.12.2005 US 751894 P; 06.11.2006 US 857034 P
(43) Date of publication of application: 03.09.2008
(73) Proprietor: THE CLEVELAND CLINIC FOUNDATION, Cleveland, OH 44195 (US)
(72) Inventor: GREENBERG, Roy, K., Bratenahl, OH 44108 (US); REZAI, Ali, R., Shaker Heights, OH 44120 (US)
(74) Representative: Naismith, Robert Stewart
(86) International application number: PCT/US2006/048283
(87) International publication number: WO 2007/075593

(56) References cited:
- WO-A-2006/041881
- US-A1- 2004 019 364

## Description

### Technical Field

The present invention generally relates to an apparatus for treating cardiovascular and nervous system disorders, and more particularly to an apparatus for modulating the baroreflex system to treat cardiovascular and nervous system disorders and their underlying causes and conditions.

### Background of the Invention

Cardiovascular disease is a major contributor to patient illness and mortality. It is also a primary driver of health care expenditure, costing more than $300 billion in 2005 in the United States (U.S.). Hypertension or high blood pressure, is a major cardiovascular disorder that is estimated to affect over 50 million people in the U.S. alone. Of those with hypertension, it is reported that fewer than 30% have their blood pressure under control. Hypertension is a leading cause of heart failure and stroke. It is the primary cause of death in over 42,000 patients per year and is listed as a primary or contributing cause of death in over 200,000 patients per year in the U.S.

A number of treatments have been proposed for the management of hypertension, heart failure, and other cardiovascular disorders. Drug treatments, for example, have been proposed and include vasodilators, diuretics, and inhibitors and blocking agents of the body's neurohormonal responses. Various surgical procedures have also been proposed for these maladies. For example, heart transplantation has been proposed for patients who suffer from severe, refractory heart failure. Alternatively, an implantable medical device such as a ventricular assist device may be implanted in the chest to increase the pumping action of the heart.

One particular method for treating hypertension, for example, includes an implantable pulse generator, carotid sinus leads, and a programmer system. A surgical implant procedure is used to place the pulse generator under the skin of a patient, near the collarbone. The electrodes are placed on the carotid arteries and the leads run under the skin and are connected to the pulse generator. The implantable pulse generator provides control and delivery of the activation energy through the carotid sinus leads, while the leads conduct activation energy from the implantable pulse generator to the left and right carotid arteries.

US Patent Application Publication No US 2004/0019364 discloses devices, systems and methods by which the blood pressure, nervous system activity, and neurohormonal activity may be selectively and controllably reduced by activating baroreceptors. A baroreceptor activation device is positioned near a baroreceptor, preferably a baroreceptor located in the carotid sinus. A control system may be used to modulate the baroreceptor activation device.

Although each of these alternative approaches is beneficial in some ways, each of the therapies has its own disadvantages. For example, drug therapy is often incompletely effective. Some patients may be unresponsive (refractory) to medical therapy. Drugs often have unwanted side effects and may need to be given in complex regimens. These and other factors contribute to poor patient compliance with medical therapy. Drug therapy may also be expensive, adding to the health care costs associated with these disorders. Likewise, surgical approaches are very costly, may be associated with significant patient morbidity and mortality, and may not alter the natural history of the disease. Accordingly, there continues to be a need for new devices and methods for treating high blood pressure, heart failure, and their associated cardiovascular and nervous system disorders.

### Summary of the Invention

In one aspect of the present invention, there is provided an apparatus for insertion into a blood vessel and for modulating the baroreflex system of a mammal according to the appended claims. The apparatus comprises an expandable support member for engaging a wall of a blood vessel at a desired location where baroreceptors are located and at least one electrode connected with the expandable support member. The at least one electrode is arranged to selectively deliver electric current to modulate the baroreceptors. The apparatus further comprises an insulative material attached to at least a portion of the expandable support member for isolating blood flowing through the vessel from the electric current delivered by the at least one electrode.

The expandable support member may be implanted intravascularly so that at least a portion of the expandable support member may be positioned substantially adjacent to a baroreceptor in the vessel wall. Electric current may be delivered to the at least one electrode to induce a baroreceptor signal to effect a change in the baroreflex system.

The apparatus may comprise first and second expandable support members. Each of the first and second expandable support members may be configured for engaging a wall of blood vessel at a desired location where baroreceptors are located. The first and second expandable support members may have at least one electrode arranged to selectively deliver electric current to modulate the baroreceptors, and an insulative material attached to at least a portion of the expandable support members for isolating blood flowing through the vessel from the electric current delivered by the at least one electrode. The first and second expandable support members may also have at least one magnetic member securely attached thereto. First and second expandable support members may be implanted at first and second intravascular locations so that the first and second expandable support members are positioned substantially adjacent from one another and so that at least a portion of each of the first and second expandable support members is positioned substantially adjacent to a baroreceptor in the vessel wall at each of the first and second intravascular locations. An electromagnetic current may be generated between the first and second expandable support members so that the at least one electrode induces a baroreceptor signal to effect a change in the baroreflex system.

The methods disclosed herein do not form part of the invention.

### Brief Description of the Drawings

The foregoing and other features of the present invention will become apparent to those skilled in the art to which the present invention relates upon reading the following description with reference to the accompanying drawings, in which:
Fig. 1 is a perspective view of an apparatus for insertion into a blood vessel and for modulating the baroreflex system constructed in accordance with the present invention;
Fig. 2 is a schematic illustration of the autonomic nervous system showing the sympathetic and parasympathetic fibers;
Fig. 3 is a schematic illustration of the upper torso of a human body showing the major arteries and veins and associated anatomy;
Fig. 4A is a cross-sectional schematic illustration of the carotid sinus and baroreceptors within the vascular wall;
Fig. 4B is a schematic illustration of baroreceptors within the vascular wall and the baroreflex system;
Fig. 5A is a perspective view showing the apparatus in Fig. 1 connected to an energy delivery source (not part of the invention);
Fig. 5B is a cross-sectional view of the apparatus shown in Fig. 5A (not part of the invention);
Fig. 6A is a perspective view showing an alternative embodiment of the apparatus shown in Fig. 1;
Fig. 6B is a cross-sectional view of the apparatus shown in Fig. 6A;
Fig. 7A is a perspective view showing another alternative embodiment of the apparatus shown in Fig. 1;
Fig. 7B is a cross-sectional view of the apparatus shown in Fig. 7A;
Fig. 8 is a perspective view showing a filter integrally formed with the apparatus in shown in Fig. 1;
Fig. 9A is a cross-sectional view of a guidewire disposed in a carotid artery;
Fig. 9B is a perspective view showing the guidewire of Fig. 9A disposed in the carotid artery;
Fig. 10A is a cross-sectional view showing the apparatus of Fig. 1 in a collapsed configuration disposed in the carotid artery;
Fig. 10B is a perspective view of the apparatus shown in Fig. 10B;
Fig. 11A is a cross-sectional view showing the apparatus in Fig. 10A in an expanded configuration in the carotid artery;
Fig. 11B is a perspective view of the apparatus show in Fig. 11A;
Fig. 12 is a cross-sectional view showing an example not forming part of the present invention disposed in a carotid artery;
Fig. 13 is a cross-sectional view showing the apparatus of Fig. 1 disposed in an external carotid artery;
Fig. 14 is a cross-sectional view showing the apparatus of Fig. 1 in an internal carotid artery;
Fig. 15 is a cross-sectional view showing an alternative embodiment of the present invention;
Fig. 16 is an enlarged view of Fig. 3 showing the apparatus of Fig. 7A and a sensor respectively implanted in the internal carotid artery and the left ventricle of the heart; and
Fig. 17 is a cross-sectional view of a heart showing the apparatus of Fig. 1 implanted in the ascending aortic arch.

### Detailed Description

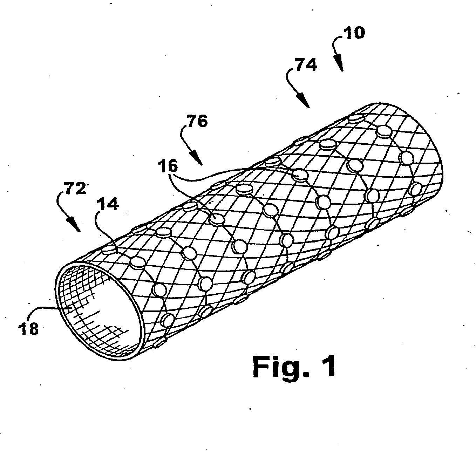

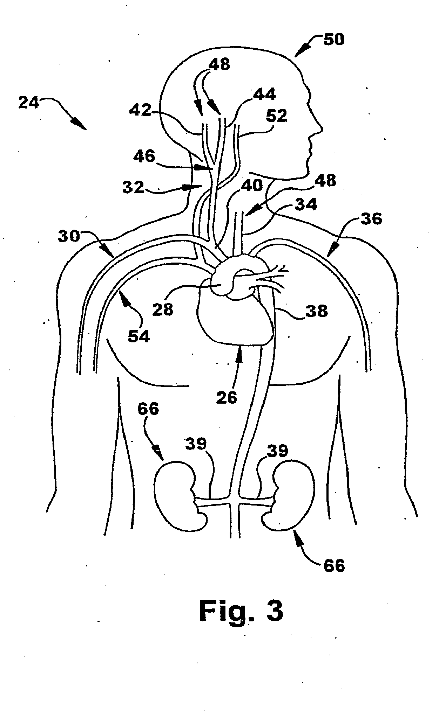

The present invention generally relates to an apparatus for treating cardiovascular and nervous system disorders, and more particularly to an apparatus for modulating the baroreflex system to treat cardiovascular and nervous system diseases, conditions, and/or functions and their underlying causes and conditions. As representative of the present invention, Fig. 1 illustrates an apparatus 10 for insertion into a blood vessel 12 (Fig. 3) comprising an expandable support member 14 (Fig. 1) having one or more electrodes 16 and an insulative material 18 attached to at least a portion of the expandable support member.

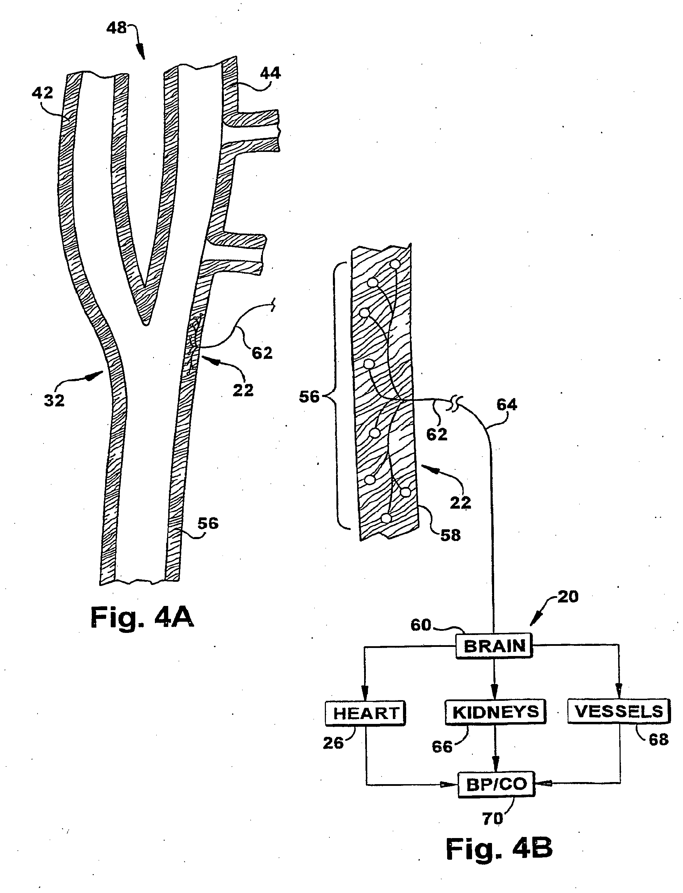

To address the problems of cardiovascular and nervous system diseases, conditions, and/or functions, the present invention provides an apparatus 10 for modulating the baroreflex system 20 (Fig. 4B) to induce a change in the autonomic nervous system. In particular, the present invention provides an apparatus 10 (Fig. 1) for modulating baroreceptors 22 (Fig. 4A) to reduce excessive blood pressure, the level of sympathetic nervous system activity, and/or neurohormonal activation. In doing so, the present invention increases parasympathetic nervous system activity and has a beneficial effect on the cardiovascular system, the nervous system, and other body systems.

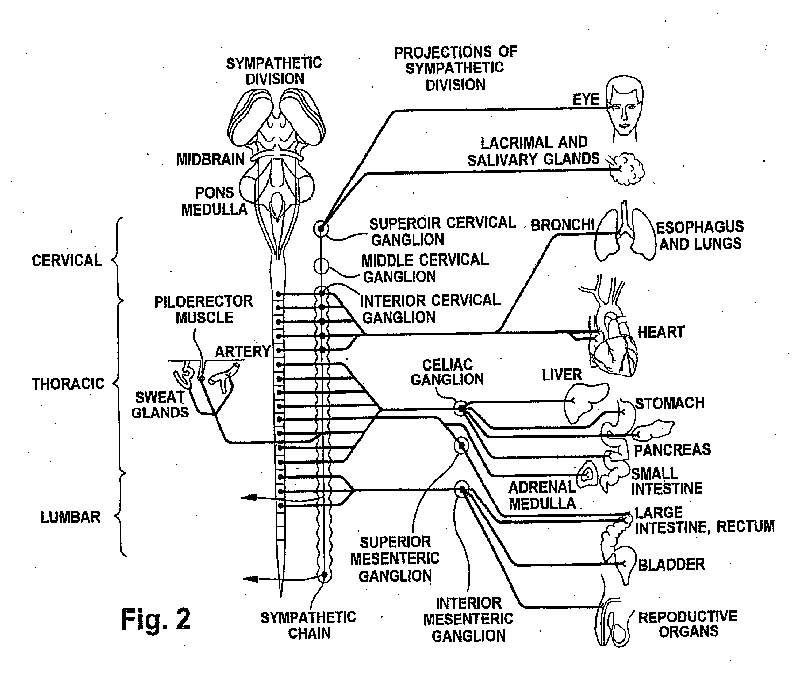

Fig. 2 is a schematic of the autonomic nervous system illustrating the sympathetic and parasympathetic fibers. The autonomic nervous system has both afferent and efferent components and hence stimulation/modulation can affect both the end organs (efferent) as well as the afferents to the brain and the central nervous system. Although sympathetic and parasympathetic fibers (axons) transmit impulses producing different effects, their neurons are morphologically similar.

The neurons of the autonomic nervous system are smallish, ovoid, multipolar cells with myelinated axons and a variable number of dendrites. All the fibers form synapses in peripheral ganglia, and the unmyelinated axons of the ganglionic neurons convey impulses to the viscera, vessels and other structures innervated. Because of this arrangement, the axons of the autonomic nerve cells in the nuclei of the cranial nerves, in the thoracolumbar lateral cornual cells, and in the gray matter of the sacral spinal segments are termed preganglionic sympathetic nerve fibers, while those of the ganglion cells are termed postganglionic sympathetic nerve fibers. These postganglionic sympathetic nerve fibers converge, in small nodes of nerve cells, called ganglia that lie alongside the vertebral bodies in the neck, chest, and abdomen. In particular, the stellate ganglion is located laterally adjacent to the intervertebral space between the seventh cervical and first thoracic vertebrae. The first, second, third and fourth thoracic ganglia lie next to their respective vertebral bodies on either side of the thoracic cavity. The effects of the ganglia as part of the autonomic system are extensive. Their effects range from the control of insulin production, blood pressure, vascular tone heart rate, sweat, body heat, blood glucose levels, sexual arousal, and digestion.

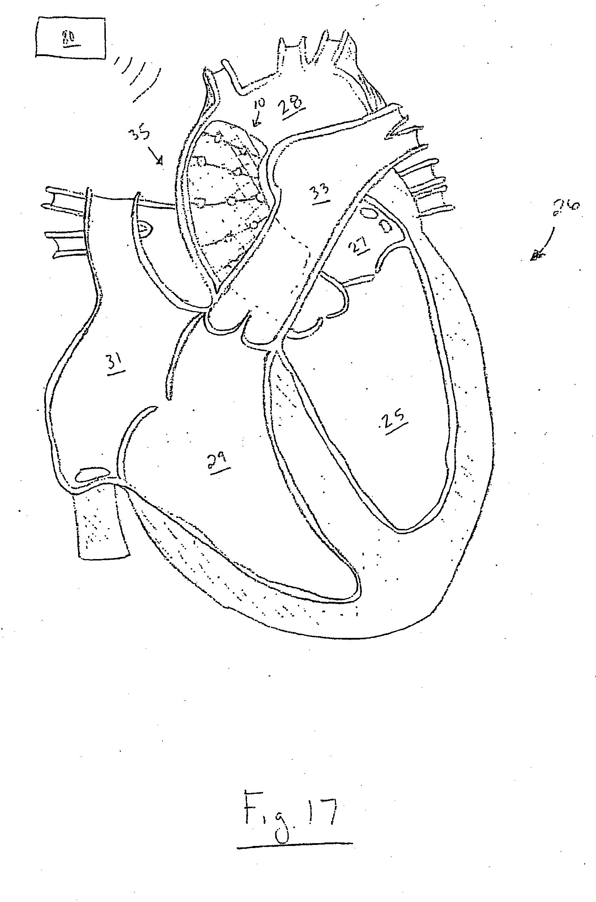

The upper torso of a human body 24 is schematically illustrated in Fig. 3. Blood from the left atrium 27 (Fig. 17) enters the left ventricle 25 of the heart 26 where oxygenated blood is pumped into the aortic arch 28. Deoxygenated blood enters the right atrium 31 from the large veins and then flows into the right ventricle 29 where the blood is pumped into the pulmonary artery 33. The right subclavian artery 30 (Fig. 3), the right common carotid artery 32, the left common carotid artery 34 and the left subclavian artery 36 branch off the aortic arch 28 proximal of the descending thoracic aorta 38. Although relatively short, a distinct vascular segment referred to as the brachiocephalic artery 40 connects the right subclavian artery 30 and the right common carotid artery 32 to the aortic arch 28. The right common carotid artery 32 bifurcates into the right external carotid artery 42 and the right internal carotid artery 44 at the right carotid sinus 46. Although not shown for purposes of clarity only, the left carotid artery 34 similarly bifurcates into the left external carotid artery (not shown) and the left internal carotid artery (not shown) at the left carotid sinus (not shown).

From the aortic arch 28, oxygenated blood flows into the carotid arteries 42 and 44 and the subclavian arteries 30 and 36. From the carotid arteries 42 and 44, oxygenated blood circulates through the head 50 and cerebral vasculature and oxygen depleted blood returns to the heart 26 by way of the jugular veins, of which only the right internal jugular vein 52 is shown for sake of clarity. From the subclavian arteries 30 and 36, oxygenated blood circulates through the upper peripheral vasculature and oxygen depleted blood returns to the heart 26 by way of the subclavian veins, of which only the right subclavian vein 54 is shown, also for sake of clarity. The heart 26 pumps the oxygen depleted blood through the pulmonary system where it is re-oxygenated. The re-oxygenated blood returns to the heart 26 which pumps the re-oxygenated blood into the aortic arch 28 as described above, and the cycle repeats.

Within the arterial walls of the aortic arch 28, common carotid arteries 32 and 34 (near the right carotid sinus 46 and left carotid sinus (not shown)), subclavian arteries 30 and 36 and brachiocephalic artery 40 there are baroreceptors 22 (Fig. 4A). For example, baroreceptors 22 reside within the vascular walls of the right carotid sinus 46. Baroreceptors 22 are a type of stretch receptor used by the body to sense blood pressure. An increase in blood pressure causes the arterial wall to stretch, and a decrease in blood pressure causes the arterial wall to return to its original size. Such a cycle is repeated with each beat of the heart 26. Because baroreceptors 22 are located within the arterial wall, they are able to sense deformation of the adjacent tissue, which is indicative of a change in blood pressure. The baroreceptors 22 located in the right carotid sinus 46, the left carotid sinus, and the aortic arch 28 play the most significant role in sensing blood pressure that affects the baroreflex system 20, which is described in more detail with reference to Fig. 4B.

Fig. 4B shows a schematic illustration of baroreceptors 22 disposed in a generic vascular wall 56 and a schematic flow chart of the baroreflex system 20. Baroreceptors 22 are profusely distributed within the arterial walls 56 of the major arteries discussed previously, and generally form an arbor 58. The baroreceptor arbor 58 comprises a plurality of baroreceptors 22, each of which transmits baroreceptor signals to the brain 60 via a nerve 62. The baroreceptors 22 are so profusely distributed and arborized within the vascular wall 56 that discrete baroreceptor arbors 58 are not readily discernable. To this end, those skilled in the art will appreciate that the baroreceptors 22 shown in Fig. 4B are primarily schematic for purposes of illustration and discussion.

Baroreceptor signals are used to activate a number of body systems which collectively may be referred to as the baroreflex system 20. Baroreceptors 22 are connected to the brain 60 via the nervous system 64. For example, the carotid sinus nerve (not shown in detail) innervates the carotid sinus. The carotid sinus is located near the bifurcation of the common carotid artery, appearing as a dilatation at the most proximal part of the internal carotid artery. It functions as a mechanoreceptor, located in the adventitia of the vessel. Changes in arterial blood pressure are sensed indirectly, through the receptor's sensitivity to mechanical deformation during vascular stretch.

Fibers from this baroreceptor form the carotid sinus nerve, which joins the glossopharyngeal nerve as it courses cranially towards the medulla oblongata. A small number of fibers reach the brain stem via the vagus and cervical sympathetic nerves. The carotid sinus nerve carries a second set of fibers which originate at the carotid body, a more complex structure located on the posterior aspect of the common carotid artery bifurcation.

The carotid body is a chemoreceptor sensitive to reductions in blood oxygen or variations in pH. The carotid body is not only passive to peripheral physiological changes; it also receives sympathetic and parasympathetic fibers that control its sensitivity to the stimuli. Changes detected by the carotid body influence the neural regulation of ventilation, thus allowing for fine adjustments in ventilation and pH regulation.

The nucleus tractus solitarius is the site for the first central synapses of the afferent fibers from the carotid sinus. Signals from the carotid sinus and from the aortic sinus (the second major baroreceptor of the major circulation) are integrated in the nucleus tractus solitarius. A common output results from this integration and is conveyed by the vagus nerves to the heart where it causes a reduction of inotropism (contractility) and chronotropism (rate). This reflex arc resulting in reduction of cardiac output and arterial blood pressure is known as the carotid baroreceptor reflex.

A severe manifestation of this reflex occurs during carotid stenting procedures. A balloon is inflated against the walls of the vessel in an attempt to increase its diameter, causing an acute and supra-physiological deformation of the arterial wall. Patients can experience an acute reduction in blood pressure and cardiac output. Asystole occurs in severe cases and may be linked to the amount of pressure applied on the arterial walls. Patients are often pre-medicated and atropine is readily utilized. An additional step for open surgeries, such as carotid endarterectmy, includes infiltration of the carotid bulb with Lidocien and mechanical denervation of the carotid to minimize this reflex.

Changes in the baroreflex system 20 allow the brain 60 to detect changes in blood pressure, which is indicative of cardiac output. If cardiac output is insufficient to meet demand (*i.e.*, the heart 26 is unable to pump sufficient blood), the baroreflex system 20 activates a number of body systems, including the heart, kidneys 66, vessels 68, and other organs/tissues. Such activation of the baroreflex system 20 generally corresponds to an increase in neurohormonal activity. Specifically, the baroreflex system 20 initiates a neurohormonal sequence that signals the heart 26 to increase heart rate and increase contraction force in order to increase cardiac output, signals the kidneys 66 to increase blood volume by retaining sodium and water, and signals the vessels 68 to constrict to elevate blood pressure. The cardiac, renal and vascular responses increase blood pressure and cardiac output 70, and thus increase the workload of the heart 26. In a patient with heart failure, this further accelerates myocardial damage and exacerbates the heart failure state.

Referring to Fig. 1, the present invention comprises an expandable support member 14 for insertion into a blood vessel 12 and for modulating the baroreflex system 20. As used herein, the term "modulate" or "modulating" refers to causing a change in neuronal activity, chemistry, and/or metabolism. The change can refer to an increase, decrease, or even a change in a pattern of neuronal activity. The term may refer to either excitatory or inhibitory stimulation, or a combination thereof, and may be at least electrical, magnetic, optical or chemical, or a combination of two or more of these. The term modulate can also be used to refer to a masking, altering, overriding, or restoring of neuronal activity.

Referring to Fig. 1, the expandable support member 14 has oppositely disposed first and second end portions 72 and 74 and a main body portion 76 extending between the end portions. The structure of the expandable support member 14 may be a mesh, a zigzag wire, a spiral wire, an expandable stent, or other similar configuration that allows the expandable support member to be collapsed and expanded. The expandable support member 14 can be comprised of a material having a high modulus of elasticity, including, for example, cobalt-nickel alloys *(e.g.*, Elgiloy), titanium, nickel-titanium alloys (*e*.*g*., Nitinol), cobalt-chromium alloys (*e.g.*, Stellite), nickel-cobalt-chromium-molybdenum alloys (*e.g.*, MP35N), graphite, ceramic, stainless steel, and hardened plastics. The expandable support member 14 may also be made of a radio-opaque material or include radio-opaque markers to facilitate fluoroscopic visualization.

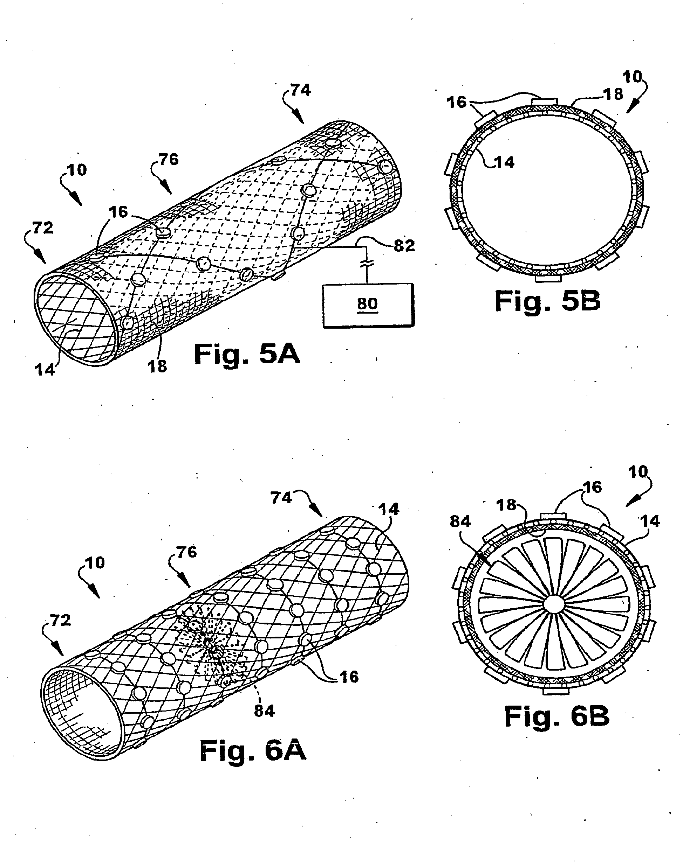

The flexible and expandable properties of the expandable support member 14 facilitate percutaneous delivery of the expandable support member, while also allowing the expandable support member to conform to a portion of the blood vessel 12. An expanded configuration of the expandable support member 14 is shown in Fig. 1. In the expanded configuration, the expandable support member 14 has a circular cross-sectional shape for conforming to the circular cross-sectional shape of the blood vessel lumen (Fig. 5B). By conforming to the shape of the blood vessel lumen, the expanded configuration of the expandable support member 14 (Fig. 1) facilitates movement of the blood flow therethrough while also maintaining lumen patency.

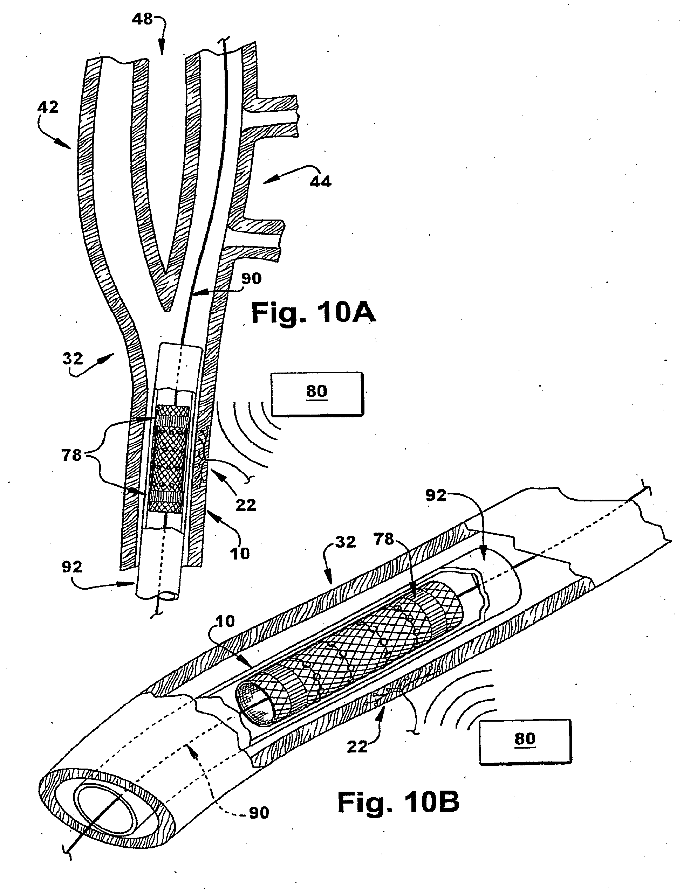

At least one constraining band 78 may be placed around the circumference of the expandable support member 14 to maintain the expandable support member in the collapsed configuration. As shown in Fig. 10A, the constraining bands 78 may comprise sutures, for example, and may be placed around the circumference of the expandable support member 14 as needed. Removal of the constraining bands 78 allows the expandable support member 14 to self-expand and obtain the expanded configuration. Where the constraining bands 78 comprise sutures, for example, the sutures may be manually broken or, alternatively, broken by the radial force generated when the expandable support member 14 self-expands. It will be appreciated that the constraining bands 78 may comprise any other type of material capable of being selectively modified. For example, the constraining bands 78 may be made of a shape memory alloy, such as Nitinol, which can be selectively modified (*i.e.*, expanded) by delivering energy (*e.g.*, thermal energy) to allow the expandable support member 14 to obtain the expanded configuration.

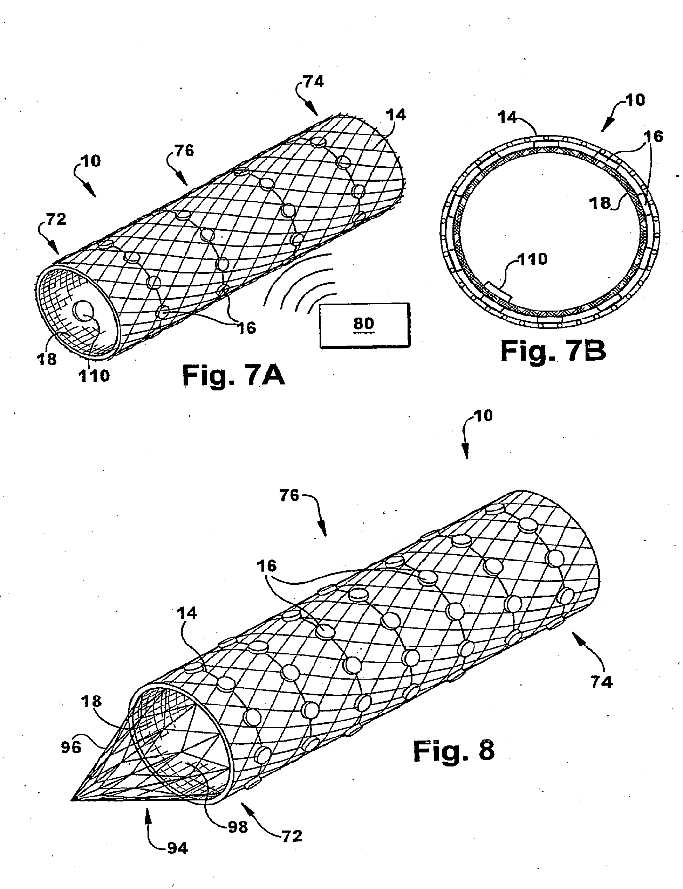

The apparatus 10 includes at least one electrode 16 for delivering an electric current to a baroreceptor 22. As shown in Fig. 1, the electrodes 16 have a flat, disc-like shape and are radially disposed about the circumference of the expandable support member 14 in a multi-electrode array configuration. It will be appreciated, however, that the electrodes 16 may have any shape and size, including, for example, a triangular shape, a rectangular shape, an ovoid shape, and/or a band-like shape (*e.g.*, a split band configuration), and are not limited to the shapes and sizes illustrated in Fig. 1. The electrodes 16 may be configured so that the apparatus 10 has a unipolar construction (Fig. 7A) using the surround tissue as ground or, alternatively, a bipolar construction using leads connected to either end of the apparatus (Fig. 5A). The electrodes 16 may be made of any material capable of conducting an electrical current, such as platinum, platinumiridium, or the like.

As shown in Fig. 1, the electrodes 16 can extend around only a portion or the entire circumference of the expandable support member 14 in a radial fashion. Alternatively, the electrodes 16 may extend around only a portion or the entire circumference of the expandable support member 14 in a sinusoidal or helical fashion (Fig. 5A). The entire length of the expandable support member 14 may be covered with the electrodes 16 or, alternatively, only a portion of the expandable support member, such as the first and second end portions 72 and 74, may be covered with the electrodes. To facilitate focal stimulation of the baroreceptors 22, the electrodes 16 may wrap around the expandable support member 14 any number of times to establish a desired electrode contact and coverage. Additionally or optionally, the entire surface area of the electrodes 16 may be conductive or, alternatively, only a portion of the surface area of the electrodes may be conductive. By modifying the conductivity of the surface of the electrodes 16, the surface area of the electrodes that touch the blood vessel wall 56 may be selectively modified to facilitate focal stimulation of the baroreceptors 22.

Electrical energy can be delivered to the electrodes 16 using a variety of internal, passive, or active energy delivery sources 80. The energy source 80 may include, for example, radio frequency (RF) energy, X-ray energy, microwave energy, acoustic or ultrasound energy such as focused ultrasound or high intensity focused ultrasound energy, light energy, electric field energy, magnetic field energy, combinations of the same, or the like. As shown in Fig. 5A, for example, an energy source 80 may be directly coupled to the apparatus 10 using an electrical lead 82. The electrical lead 82 may be disposed in an adjacent blood vessel 12, such as the jugular vein 52, and travel down the length of the jugular vein to a remote entry site (not shown). Alternatively, as shown in Fig. 6A, electrical energy may be supplied to the electrodes 16 via a turbine-like mechanism 84 operatively disposed in the lumen of the expandable support member 14. As blood flows through the lumen of the expandable support member 14, the turbine mechanism 84 generates electrical energy which may then be delivered to the electrodes 16. Further, the energy source 80 may be wirelessly coupled to the apparatus 10 as shown in Fig. 7A.

Electrical energy can be delivered to the electrodes 16 continuously, periodically, episodically, or a combination thereof. For example, electrical energy can be delivered in a unipolar, bipolar, and/or multipolar sequence or, alternatively, via a sequential wave, charge-balanced biphasic square wave, sine wave, or any combination thereof. Electrical energy can be delivered to all the electrodes 16 at once or, alternatively, to only a select number of desired electrodes. The particular voltage, current, and frequency delivered to the electrodes 16 may be varied as needed. For example, electrical energy can be delivered to the electrodes 16 at a constant voltage *(e.g.,* at about 0.1 v to about 25 v), at a constant current *(e.g.,* at about 25 microampes to about 50 milliamps), at a constant frequency (*e.g.*, at about 5 Hz to about 10,000 Hz), and at a constant pulse-width (*e.g.*, at about 50 µsec to about 10,000 µsec).

Delivery of electrical energy to a select number of electrodes 16 may be accomplished via a controller (not shown), for example, operably attached to the apparatus 10. The controller may comprise an electrical device which operates like a router by selectively controlling delivery of electrical energy to the electrodes 16. For example, the controller may vary the frequency or frequencies of the electrical energy being delivered to the electrodes 16. By selectively controlling delivery of electrical energy to the electrodes 16, the controller can facilitate focal stimulation of the baroreceptors 22.

Typically, delivery of electrical energy to the apparatus 10 results in activation of the baroreceptors 22. Alternatively, deactivation or modulation of the apparatus 10 may cause or modify activation of the baroreceptors 22. For example, electrical energy may be delivered to the electrodes 16 to inhibit baroreceptor 22 activation by hyperpolarizing cells in or adjacent to the baroreceptors. Modulating the baroreceptors 22 may induce a change or changes in the activity, chemistry, and/or metabolism of the nerve(s) directly or indirectly associated with the baroreceptors.

Examples of nerves associated with baroreceptors 22 include the nerves illustrated in Fig. 2, as well as, but not limited to, the cardiac nerve, the internal carotid nerve, the vagus nerve, the glossopharyngeal nerve, the trigeminal nerve, the facial nerve, the vestibulo-cochlear nerve, the mandibular nerve, the superior laryngeal nerve, the deep petrosal nerve, the nerve of the pterygoid canal, the oculomotor nerve, the maxiliary nerve, the ophthalmic nerve, the nasociliary nerve, the lingual nerve, the inferior alveolar nerve, the sympathetic trunk, the gray rami communicantes, the pharyngeal plexus, and the phrenic nerve.

Other examples include parasympathetic nerve and ganglia such as the cardiac and pulmonary plexus, celiac plexus, hypogastric plexus, pelvic nerves, and/or sympathetic nerve and ganglia such as the cervical sympathetic ganglia, spinal nerves (dorsal and ventral rami), postganglionic fibers to spinal nerves (innervating skin, blood vessels, sweat glands, erector pili muscle, adipose tissue), sympathetic chain ganglia, coccygeal ganglia, cardiac and pulmonary plexus, greater splanchnic nerve, lesser splanchnic nerve, inferior mesenteric ganglion, celiac ganglion, superior mesenteric ganglion and lumber splanchnic nerves. Still further examples of nerves that may be modulated via the baroreceptors 22 will be understood by those skilled in the art.

It should be appreciated, however, that means other than electrical energy, such as chemical or biological means, may also be used to modulate the baroreflex system 20. For example, the apparatus 10 may include at least one therapeutic agent for eluting into the vascular tissue and/or blood stream. The therapeutic agent may be capable of preventing a variety of pathological conditions including, but not limited to, hypertension, hypotension, arrhythmias, thrombosis, stenosis and inflammation. Accordingly, the therapeutic agent may include at least one of an anti-arrhythmic agent, an anti-hypertensive, an anti-hypotensive agent, an anticoagulant, an antioxidant, a fibrinolytic, a steroid, an anti-apoptotic agent, and/or an anti-inflammatory agent.

Optionally or additionally, the therapeutic agent may be capable of treating or preventing other diseases or disease processes such as microbial infections and heart failure. In these instances, the therapeutic agent may include an inotropic agent, a chronotropic agent, an anti-microbial agent, and/or a biological agent such as a cell, peptide, or nucleic acid. The therapeutic agent can be simply linked to the surface of the apparatus 10, embedded and released from within polymer materials, such as a polymer matrix, or surrounded by and released through a carrier.

Referring again to Fig. 1, the apparatus 10 additionally comprises an insulative material 18 for isolating blood flow through the vessel 12 from the electric current. More particularly, the insulative material 18 serves as an electrical insulator, separating electrical energy from blood flow and facilitating delivery of the electrical energy to the vessel wall 56. The insulative material 18 is disposed radially inward of the electrodes 16 and extends along the entire length of the expandable support member 14. The insulative material 18 is disposed about the lumen of the expandable support member (Figs. 6B and 7B). The insulative material 18 generally has a low electrical conductivity and a non-thrombogenic surface. The insulative material 18 can include materials such as PTFE, ePTFE, silicone, silicone-based materials, and the like.

In addition to the insulative layer 18, at least a portion of the apparatus 10 may optionally include a layer (not shown) of biocompatible material. The layer of biocompatible material may be synthetic such as Dacron^{®} (Invista, Wichita, KS), Gore-Tex^{®} (W. L. Gore & Associates, Flagstaff, AZ), woven velour, polyurethane, or heparin-coated fabric. Alternatively, the layer of biocompatible material may be a biological material such as bovine or equine pericardium, peritoneal tissue, an allograft, a homograft, patient graft, or a cell-seeded tissue. The biocompatible layer can cover either the luminal surface of the expandable support member 14, the non-luminal surface of the expandable support member, or can be wrapped around both the luminal and non-luminal surfaces. The biocompatible layer may be attached around the entire circumference of the expandable support member 14 or, alternatively, may be attached in pieces or interrupted sections to allow the expandable support member to more easily expand and contract.

As shown in Fig. 8, the apparatus 10 may additionally include a filter 94 sized to allow blood flow through the expandable support member 14 while retaining obstructive material. The filter 94 may be integrally formed with the first end portion 72 of the expandable support member 14 or, alternatively, the filter may be integrally formed with the second end portion 74 of the expandable support member. Further, the filter 94 may be integrally formed with the first and second end portion 72 and 74 of the expandable support member 14.

The filter 94 may have any configuration that allows blood to flow freely through the expandable support member 14 while also trapping all or a portion of an obstructive material, such as an obstructing thrombus, clot, or other cellular debris, that may have been dislodged or fragmented during deployment of the apparatus 10. As shown in Fig. 7, for example, the filter 94 has a conical shape and is made of a plurality of wire segments 96 which form interstices 98 that allow blood flow through the expandable support member 14 while retaining all or a portion of an obstructing material. It should be appreciated that the filter 94 can have any other suitable shape (*e.g.*, tubular, convex, concave) and be made of any material (*e.g.*, wire mesh, ePTFE, stainless steel, etc.) appropriate for retaining and/or fragmenting obstructive material. It should be further appreciated that the configuration of the filter 94 illustrated in Fig. 7 is not intended to be limiting and, rather, that the filter 94 may comprise any one or combination of other configurations known in the art. For example, the filter 94 may be reconstrainable and/or entirely removable from the apparatus 10.

The present invention can be used in a method for modulating the baroreflex system 20 of a mammal. As used herein, the term "mammal" refers to any warm-blooded organism including, but not limited to, human beings, rats, mice, dogs, goats, sheep, horses, monkeys, apes, rabbits, cattle, etc. According to one method an apparatus 10 is implanted intravenously so that at least a portion of the expandable support member 14 is positioned substantially adjacent to a baroreceptor 22 in a vessel wall. The baroreceptor 22 can include both high and low pressure baroreceptors. High pressure baroreceptors are typically present in the aortic arch 28 and the carotid arteries 48, while low pressure baroreceptors are typically present in the vasculature beyond the aortic arch 28 and the carotid arteries 48, such as the walls of the atria (not shown in detail) and the large veins (not shown).

The apparatus 10 is positioned substantially adjacent to a baroreceptor 22 at a desired location, such as an arterial or a venous wall. Examples of suitable arterial wall locations include, without limitation, a carotid arterial wall, an aortic arterial wall, a subclavian arterial wall, a brachiocephalic arterial wall, a renal arterial wall, a hepatic arterial wall, a splenic arterial wall, a pancreatic arterial wall, a jugular arterial wall, a femoral arterial wall, an iliac arterial wall, a pulmonary arterial wall, a brachial arterial wall, a cardiac arterial wall, a popliteal arterial wall, a tibial arterial wall, a celiac arterial wall, an axillary arterial wall, a radial arterial wall, an ulnar arterial wall, and a mesenteric arterial wall.

Examples of suitable venous wall locations include, without limitation, a hepatic venous wall, an inferior vena cava venous wall, a superior vena cava venous wall, a jugular venous wall, a subclavian venous wall, an iliac venous wall, a femoral venous wall, a pulmonary venous wall, a splenic venous wall, a renal venous wall, a pancreatic venous wall, a cephalic venous wall, a tibial venous wall, an axillary venous wall, a brachial venous wall, a popliteal venous wall, a cardiac venous wall, and a brachiocephalic venous wall.

Placement of the apparatus 10 substantially adjacent to a baroreceptor 22 in a vessel wall permits treatment of a disease or condition by selectively delivering electric current to the electrodes 16 to induce a baroreceptor signal and effect a change in the baroreflex system 20. For example, by placing the apparatus 10 in the pulmonary artery (not shown) (*e.g.*, near the ligamentum arteriosum (not shown) and/or the trunk of the pulmonary artery) of a human patient, electrical energy may be selectively delivered to the electrodes 16 to treat bradyarrhythmia, tachyarrhythmia, and/or congestive heart failure, for example. Additionally or alternatively, the apparatus 10 may be placed in a renal artery (not shown) (*e.g.*, in an afferent renal arteriole) to treat a renal disease or condition including, for example, insufficient renovascular perfusion, renal failure, and/or renal hypertension. Other examples of diseases, conditions, or functions which may be treated using the present invention include, but are not limited to, chronic pain and/or headaches, respiratory, hepatic, cerebrovascular (*e.g.*, cerebral vasospasm), cardiac, gastrointestinal, genitourinary, pancreatic, splenic, neurological, skeletal, immunological, muscular or connective, ocular, auditory or vestibular, olfactory, dermatological, endocrinological, reproductive, psychological, neoplastic, and/or inflammatory diseases, conditions or functions.

According to another method an apparatus 10 is implanted in a blood vessel 12 to modulate the baroreflex system 20 of a human patient having, for example, a disease characterized by high blood pressure (*e.g.*, hypertension). The apparatus 10 is implanted using a minimally invasive, percutaneous, or endovascular approach. It should be appreciated, however, that a minimally invasive surgical approach may also be used. The apparatus 10, or only a portion of the apparatus, is positioned immediately adjacent the baroreceptors 22 in a blood vessel, such as the right common carotid artery 32, the right internal carotid artery 44, the right external carotid artery 42, the aortic arch 28, the right subclavian artery 30, or the brachiocephalic artery 40. For purposes of illustration only, the method is described with reference to the apparatus 10 being positioned in the right common carotid artery 32.

Prior to use of the apparatus 10, the dimensions of the right common carotid artery 32, including the right carotid sinus 46 near the origin of the right internal carotid artery 44, will need to be determined. Various methods and devices for determining the dimensions of the right common carotid artery 32 are known in the art, including, for example, computed tomography, magnetic resonance imaging, angiography and fluoroscopy. After determining the dimensions of the right common carotid artery 32, an appropriately-sized apparatus 10 is chosen. The apparatus 10 is suitably sized, for example, so that the dimensions of the apparatus in the expanded configuration correspond to the dimensions of the right common carotid artery 32.

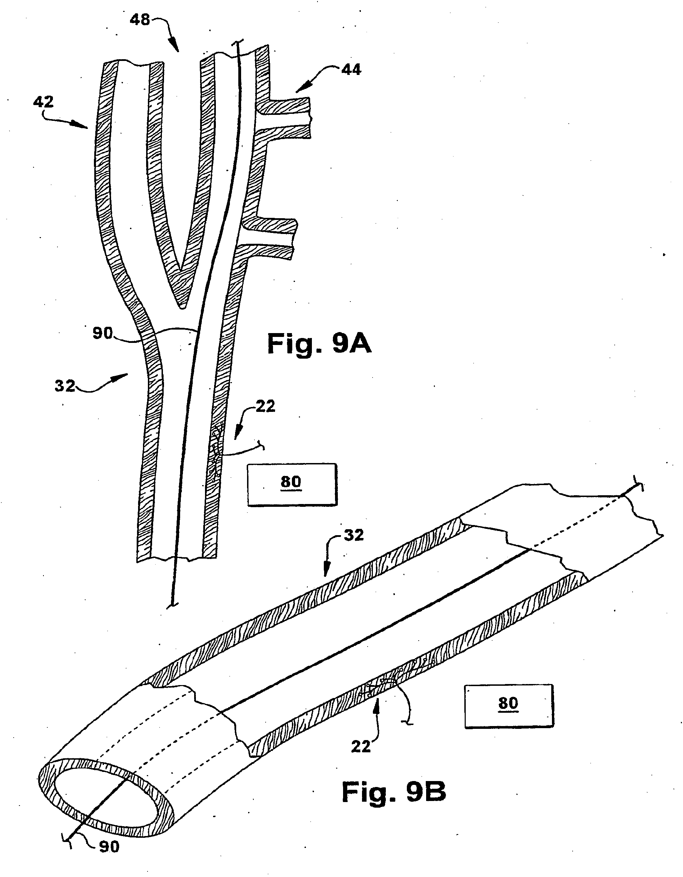

Percutaneous placement of the apparatus 10 starts by accessing a bodily vessel 12 with a delivery device 88. For instance, a guidewire 90 may be introduced into the vasculature of the patient via a vascular opening (not shown). Vascular access may be through a peripheral arterial access site (not shown), such as the femoral artery (not shown). The guidewire 90 is inserted through the incision into the right brachiocephalic artery 40 in an antegrade direction. Alternatively, the guidewire 90 may be inserted into the brachiocephalic artery 40 from an incision in the left subclavian artery 36 or left brachial artery (not shown) in a retrograde direction, or in a retrograde direction through the right subclavian artery 30. The guidewire 90 is then urged into the right common carotid artery 32 and the right internal carotid artery 44 as shown in Figs. 9A and 9B.

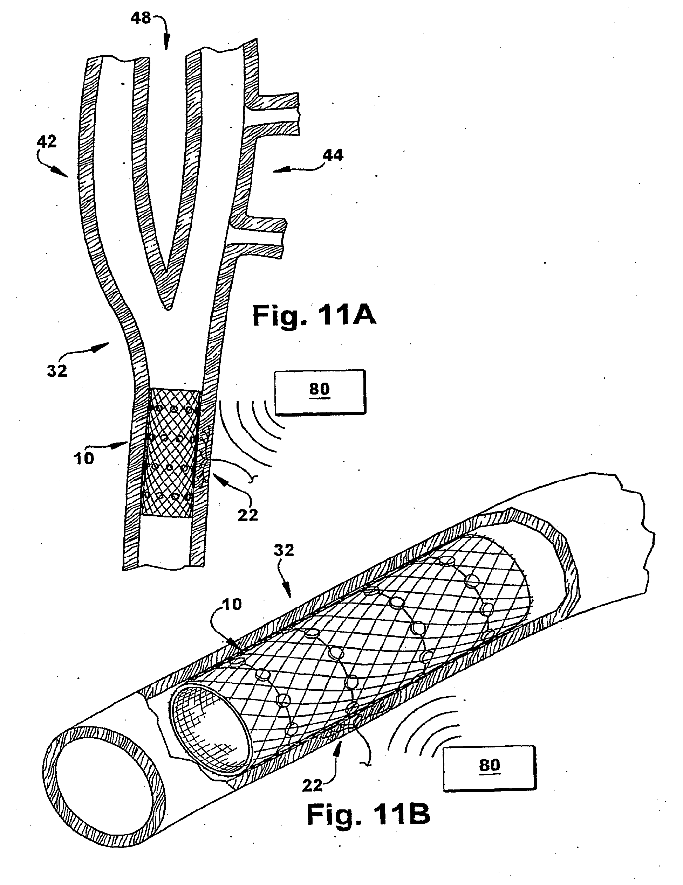

Next, the apparatus 10 is placed in a delivery catheter 92 in a collapsed configuration and securely attached to a proximal end (not shown) of the guidewire 90. The delivery catheter 92 is then advanced over the guidewire 90 until the delivery catheter is suitably positioned in the right common carotid artery 32 as shown in Figs. 10A and 10B. Once the apparatus 10 is appropriately positioned in the right common carotid artery 32, the delivery catheter 92 is removed and the constraining bands 78 are progressively released (*i.e.*, broken) by the radial force generated by the self-expanding expandable support member 14. When all of the constraining bands 78 have been released, the expandable support member 14 obtains the expanded configuration and the apparatus 10 is securely positioned in the right common carotid artery 32 (Figs. 11A and 11B). With the apparatus 10 securely positioned in the right common carotid artery 32, the guidewire 90 may then be removed from the vasculature of the patient.

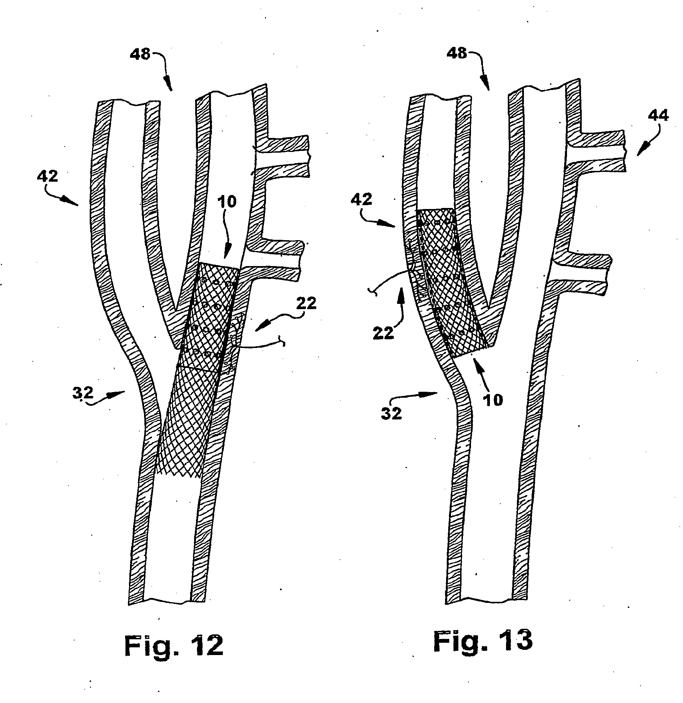

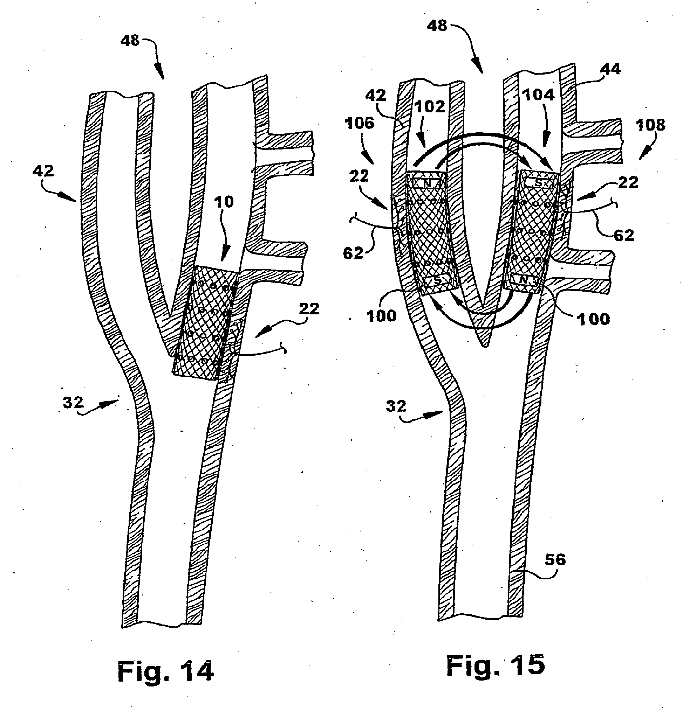

It should be appreciated that alternative methods may be used to deliver the apparatus 10 to a desired location. For example, once the apparatus 10 is appropriately positioned in a blood vessel 12, the apparatus may be expanded via an inflatable balloon (not shown) or, alternatively, via delivery of thermal energy (*e.g.*, where the constraining bands 78 are comprised of a shape memory alloy). It should also be appreciated that the apparatus 10 may be placed in a blood vessel 12 in a position other than the one illustrated in Figs. 11A and 11B. For example, the first and second end portions 72 and 74 of the apparatus 10 may be respectively positioned in the right common carotid artery 32 and right internal carotid artery 44 (Fig. 12). Alternatively, the apparatus 10 may be positioned in the right external carotid artery 42 (Fig. 13), the right internal carotid artery 44 (Fig. 14), the ascending aortic arch 35 (Fig. 17),or the descending thoracic aorta 38 near the left subclavian artery 36 (not shown).

After the guidewire 90 has been removed from the patient, electrical energy is delivered to the apparatus 10. As shown in Figs. 11A and 11B, for example, RF energy may be delivered to the apparatus 10 via a wirelessly coupled energy delivery source 80. As electrical energy is delivered to the apparatus 10, the electrodes 16 conduct the electrical energy to the vascular wall 56 and activate the baroreceptors 22 by causing the baroreceptors to fire action potentials. The action potentials are then relayed to the nucleus of the tractus solitarius (not shown), which uses spike frequency as a surrogate measure of blood pressure. Increased activation of the tractus solitarius inhibits the vasomotor center (not shown) and stimulates the vagal nuclei (not shown). Consequently, the activity of the sympathetic nervous system is reduced or inhibited, the activity of the parasympathetic nervous system is activated or increased, and the blood pressure of the patient is successfully decreased to treat hypertension.

It should be appreciated that by selectively activating, deactivating or otherwise modulating the electrical energy transmitted to the apparatus 10, one or more baroreceptors 22 may be directly activated by changing the electrical potential across the baroreceptors. It is also possible that changing the electrical potential might indirectly change the thermal or chemical potential across the tissue surrounding the baroreceptors 22 and/or otherwise may cause the surrounding tissue to stretch or otherwise deform, thus mechanically activating the baroreceptors. It is also contemplated that the electrodes 16 may activate the baroreceptors 22 by delivering thermal energy. For example, thermal energy may be delivered by utilizing a semi-conductive material having a high resistance such that the electrodes 16 resistively generate heat upon application of electrical energy.

During delivery of electrical energy to the apparatus 10, at least one metabolic parameter of interest, such as the blood pressure of the patient may be monitored by, for example, a blood pressure cuff (not shown) (or similar device) or, alternatively, via a sensor 110 (Fig. 7A). The sensor 110 may comprise any suitable device that measures or monitors a parameter indicative of the need to modify the activity of the apparatus 10. For example, the sensor 110 may comprise a physiologic transducer or gauge that measures ECG, blood pressure (systolic, diastolic, average or pulse pressure), blood volumetric flow rate, blood flow velocity, blood pH, O₂ or CO₂ content, nitrogen content, respiratory rate and/or respiratory efficiency, hemodynamic factors (*e.g.*, renin/angiotensin, blood glucose, inflammatory mediators, cardiac enzymes, tissue factors, etc.), mixed venous oxygen saturation (SVO₂), vasoactivity, nerve activity, and tissue activity or composition.

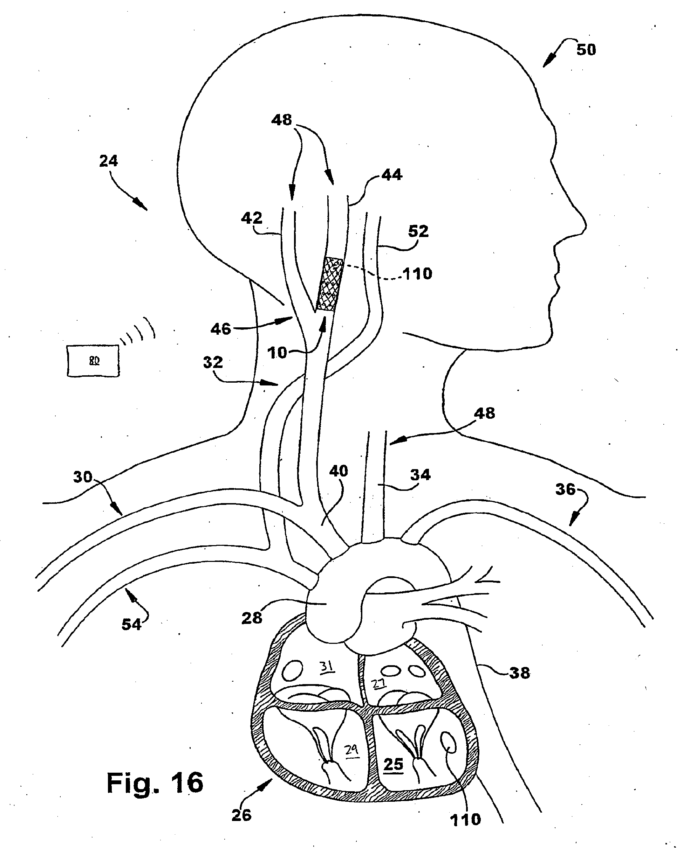

The sensor 110 may be separate from the apparatus 10 or combined therewith (Fig. 7A). The sensor 110 may also be positioned in/on a blood vessel 68 and/or organ, such as in a chamber of the heart 26 or in/on a major artery such as the aortic arch 28, a common carotid artery 32 and 34, a subclavian artery 30 and 36, or the brachiocephalic artery 40 such that the parameter of interest may be readily ascertained. As shown in Fig. 16, for example, a first sensor 110 may be combined with the apparatus 10 and a second sensor positioned in the left ventricle 25 of the heart 26.

An electrical stimulus regimen comprising a desired temporal and spatial distribution of electrical energy to the baroreceptors 22 of the patient may be selected to promote long term efficacy of the present invention. It is theorized that uninterrupted or otherwise unchanging activation of the baroreceptors 22 may result in the baroreceptors and/or the baroreflex system 20 becoming less responsive over time, thereby diminishing the long term effectiveness of the therapy. Therefore, the electrical stimulus regimen maybe selected to activate, deactivate or otherwise modulate the apparatus 10 in such a way that therapeutic efficacy is maintained for a desired period of time.

In addition to maintaining therapeutic efficacy over time, the electrical stimulus regimen may be selected to reduce the power requirement/consumption of the present invention. For example, the electrical stimulus regimen may dictate that the apparatus 10 be initially activated at a relatively higher energy and/or power level, and then subsequently activated at a relatively lower energy and/or power level. The first level attains the desired initial therapeutic effect, and the second (lower) level sustains the desired therapeutic effect long term. By reducing the energy and/or power levels after the desired therapeutic effect is initially attained, the energy required or consumed by apparatus 10 is also reduced long term.

It should be appreciated that unwanted collateral stimulation of adjacent tissues may be limited by creating localized cells or electrical fields (*i.e.*, by limiting the electrical field beyond the vascular wall 56 where the baroreceptors 22 reside). Localized cells may be created by, for example, spacing the electrodes 16 very close together or biasing the electrical field with conductors and/or magnetic fields. For example, electrical fields may be localized or shaped by using electrodes 16 with different geometries, by using one or more multiple electrodes, and/or by modifying the frequency, pulse-width, voltage, stimulation waveforms, paired pulses, sequential pulses, and/or combinations thereof.

It should also be appreciated that more than one apparatus 10 may be used to modulate the baroreflex system 20 of a patient. For example, it may be desirable to modulate the carotid sinus nerve via an electrical field by placing one apparatus 10 in the right external carotid artery 42 and another apparatus in the right internal carotid artery 44. Alternatively, one apparatus 10 may be placed in the right external carotid artery 42 and another apparatus placed in the jugular vein 52, etc. With this arrangement, the electrical field created between the two apparatuses 10 may be used to modulate the carotid sinus nerve for baropacing applications. Additionally or optionally, the electrical field created between the two apparatuses 10 may be used to modulate the circadian rhythm and/or postural changes.

In another method, the baroreflex system 20 of a mammal is modulated. The method comprises implanting first and second expandable support members 102 and 104 (Fig. 15) at first and second intravascular locations 106 and 108 so that at least a portion of each of the first and second expandable support members is positioned substantially adjacent to a baroreceptor 22 in a vessel wall at each of the intravascular locations. The first and second expandable support members 102 and 104 are identically constructed as the expandable support member 14 in Fig. 1, except where as described below.

As shown in Fig. 15, each of the first and second expandable support members 102 and 104 may have one or more electrodes 16 arranged to selectively deliver electric current to modulate baroreceptors 22 in a vessel wall. Each of the first and second expandable support members 102 and 104 may also have an insulative material 18 attached to at least a portion of each of the expandable support members for isolating blood flowing through the vessel from an electric current. Additionally or optionally, each of the first and second expandable support members 102 and 104 may include a filter 94 that allows blood to flow freely through the expandable support members while also trapping all or a portion of an obstructive material that may have been dislodged or fragmented during deployment of the expandable support members.

At least one magnetic member 100 may also be securely attached to each of the first and second expandable support members 102 and 104. The magnetic member 100 may be made of any one or combination of known electromagnetic materials including, for example, iron, NdFeB, SmCO and Alnico. The magnetic member 100 may be securely attached to the first and second expandable support members 102 and 104 using any suitable means known in the art including, for example, soldering, staples, clips, sutures, and/or adhesives. The magnetic member 100 may be attached to the first and second expandable support members 102 and 104 at any desired location, such as at the first and second end portion 72 and 74 of each of the expandable support members. The magnetic member 100 may have any suitable shape or configuration including, for example, a circular shape, an ovoid shape, a square-like shape, and/or a rectangular shape. Alternatively or additionally, the magnetic member 100 may have a ring-like shape to completely encircle each of the first and second expandable support members 102 and 104

The first and second expandable support members 102 and 104 may be implanted at the first and second intravascular locations 106 and 108, respectively, using a minimally invasive, percutaneous, or endovascular approach. The first and second expandable support members 102 and 104 may be respectively implanted in an arterial and venous vessel, in first and second arterial vessels, and/or in first and second venous vessels. For example, the first and second expandable support members 102 and 104 may be respectively implanted in a carotid artery 48 and a jugular vein 52.

As illustrated in Fig. 15, first and second expandable support members 102 and 104 may be implanted substantially adjacent to baroreceptors 22 in the right external carotid artery 42 and the right internal carotid artery 44, respectively. As described above, the first and second expandable support members 102 and 104 may be implanted using a percutaneous approach. After the first and second expandable support members 102 and 104 are securely positioned substantially adjacent to the baroreceptors 22 in each artery 42 and 44, an electromagnetic force may be generated between the magnetic members 100 of the first and second expandable support members. As indicted by the directional lines in Fig. 15, the magnetic members 100 are polarized upon placement and consequently generate an electric current between one another. The electric current may then be distributed across the electrodes 16 of the first and second expandable support members 102 and 104 and then delivered to the baroreceptors 22 of the right external and internal carotid arteries 42 and 44, respectively, to modulate the baroreflex system 20 of the patient. By adjusting the size, number, and composition of the magnetic members 100, in addition to the position of the first and second expandable support members 102 and 104, the magnitude and direction of the electric current may be varied as needed.

Although the aforementioned embodiments of the present invention have been described with respect to modulating the baroreflex system 20, the present invention also provides devices for transvascularly modulating other target sites in the body 24 as well. Such target sites can include target sites in the neurological, cardiovascular, gastrointestinal, endocrinological, respiratory or pulmonary, reproductive, urinary, skeletal, renal, muscular or connective, vascular or hematological, ocular, olfactory, auditory or vestibular, and dental bodily systems. Specific, exemplary target sites within these bodily systems are provided in US-A-2006/085 046 and US-A-2006/111 754.

From the above description of the invention, those skilled in the art will perceive improvements, changes and modifications. For example, to address hypotension and other conditions requiring blood pressure augmentation, the present invention may be used to selectively and controllably regulate blood pressure by inhibiting or dampening or modulating baroreceptor 22 signals. Specifically, the present invention may increase the blood pressure and level of sympathetic nervous system activation by inhibiting or dampening the activation of baroreceptors 22.

## Claims

1. An apparatus (10) for insertion into a blood vessel (12) and for modulating the baroreflex system of a mammal, said apparatus comprising:
an expandable support member (14) for engaging a wall of a blood vessel at a desired location where baroreceptors (22) are located said expandable support member including a lumen, said expandable support member being defined by an inner circumferential surface and an outer circumferential surface;
an insulative material (18) attached to at least a portion of said expandable support member, said insulative material extending the entire longitudinal length of said expandable support member, said insulative material extending circumferentially about the entirety of said lumen of said expandable support member; and
at least one electrode (16) connected with said expandable support member and arranged to selectively deliver electric current to modulate the baroreceptors;
said insulative material being arranged for isolating blood flowing through the vessel from the electric current delivered by said at least one electrode.

2. The apparatus of claim 1, wherein said expandable support member further comprises a filter (94) sized to allow blood flow through said expandable support member while retaining obstructive material, said filter being integrally formed with said expandable support member.

3. The apparatus of claim 1, wherein at least one magnetic member (100), is securely attached to said expandable support member.

4. The apparatus of claim 1, wherein said insulative material is connected to said inner circumferential surface of said expandable support member.

5. The apparatus of claim 1 wherein said at least one electrode is connected with said outer circumferential surface of said expandable support member.

6. The apparatus of claim 1, wherein said at least one electrode is connected with said inner circumferential surface of said expandable support member.

7. The apparatus of claim 6, wherein said at least one electrode is connected with said insulative layer.

8. The apparatus of claim 7, wherein at least one sensor (110) for measuring at least one metabolic parameter of interest is attached to said insulative material.

## Patentansprüche

1. Vorrichtung (10) zum Einsetzen in ein Blutgefäß (12) und zur Modulation des Baroreflex-Systems eines Säugers, wobei die Vorrichtung aufweist:
ein expandierbares Stützelement (14) für einen Eingriff mit einer Wand eines Blutgefäßes an einer gewünschten Stelle, wo Barorezeptoren (22) angeordnet sind, wobei das expandierbare Stützelement ein Lumen umfasst, wobei das expandierbare Stützelement durch eine innere Umfangsfläche und eine äußere Umfangsfläche definiert wird;
ein isolierendes Material (18), das an mindestens einem Abschnitt des expandierbaren Stützelementes befestigt ist, wobei sich das isolierende Material über die gesamte Länge in der Längsrichtung des expandierbaren Stützelementes erstreckt, wobei sich das isolierende Material peripher um das gesamte Lumen des expandierbaren Stützelementes erstreckt; und
mindestens eine Elektrode (16), die mit dem expandierbaren Stützelement verbunden und angeordnet ist, um selektiv einen elektrischen Strom zu liefern, um die Barorezeptoren zu modulieren,
wobei das isolierende Material angeordnet ist, damit das Blut, das durch das Gefäß fließt, vom elektrischen Strom isoliert wird, der durch die mindestens eine Elektrode geliefert wird.

2. Vorrichtung nach Anspruch 1, bei der das expandierbare Stützelement außerdem einen Filter (94) aufweist, der bemessen ist, um einen Blutfluss durch das expandierbare Stützelement zu gestatten, während verstopfendes Material zurückgehalten wird, wobei der Filter zusammenhängend mit dem expandierbaren Stützelement ausgebildet ist.

3. Vorrichtung nach Anspruch 1, bei der mindestens ein magnetisches Element (100) sicher am expandierbaren Stützelement befestigt wird.

4. Vorrichtung nach Anspruch 1, bei der das isolierende Material mit der inneren Umfangsfläche des expandierbaren Stützelementes verbunden ist.

5. Vorrichtung nach Anspruch 1, bei der die mindestens eine Elektrode mit der äußeren Umfangsfläche des expandierbaren Stützelementes verbunden ist.

6. Vorrichtung nach Anspruch 1, bei der die mindestens eine Elektrode mit der inneren Umfangsfläche des expandierbaren Stützelementes verbunden ist.

7. Vorrichtung nach Anspruch 6, bei der die mindestens eine Elektrode mit der isolierenden Schicht verbunden ist.

8. Vorrichtung nach Anspruch 7, bei der mindestens ein Sensor (110) für das Messen von mindestens einem interessierenden metabolischen Parameter am isolierenden Material befestigt ist.

## Revendications

1. Appareil (10) destiné à être inséré dans un vaisseau sanguin (12) et permettant une modulation du système baroréflexe d'un mammifère, ledit appareil comprenant :
un élément de soutien expansible (14) permettant de mettre en prise une paroi d'un vaisseau sanguin au niveau d'un emplacement souhaité où sont situés des barorécepteurs (22), ledit élément de soutien expansible comprenant une lumière, ledit élément de soutien expansible étant défini par une surface circonférentielle intérieure et une surface circonférentielle extérieure ;
un matériau isolant (18) fixé à au moins une partie dudit élément de soutien expansible, ledit matériau isolant s'étendant sur l'intégralité de la longueur longitudinale dudit élément de soutien expansible, ledit matériau isolant s'étendant circonférentiellement autour de l'intégralité de ladite lumière dudit élément de soutien expansible ; et
au moins une électrode (16) raccordée audit élément de soutien expansible et agencée pour fournir de manière sélective du courant électrique afin de moduler les barorécepteurs ;
ledit matériau isolant étant agencé pour isoler le sang s'écoulant dans le vaisseau du courant électrique fourni par ladite au moins une électrode.

2. Appareil selon la revendication 1, dans lequel ledit élément de soutien expansible comprend en outre un filtre (94) dimensionné de manière à permettre un écoulement de sang à travers ledit élément de soutien expansible tout en retenant de la matière obstructive, ledit filtre étant formé d'un seul tenant avec ledit élément de soutien expansible.

3. Appareil selon la revendication 1, dans lequel au moins un élément magnétique (100) est solidement fixé audit élément de soutien expansible.

4. Appareil selon la revendication 1, dans lequel ledit matériau isolant est raccordé à ladite surface circonférentielle intérieure dudit élément de soutien expansible.

5. Appareil selon la revendication 1, dans lequel ladite au moins une électrode est raccordée à ladite surface circonférentielle extérieure dudit élément de soutien expansible.

6. Appareil selon la revendication 1, dans lequel ladite au moins une électrode est raccordée à ladite surface circonférentielle intérieure dudit élément de soutien expansible.

7. Appareil selon la revendication 6, dans lequel ladite au moins une électrode est raccordée à ladite couche isolante.

8. Appareil selon la revendication 7, dans lequel au moins un capteur (110) permettant de mesurer au moins un paramètre métabolique d'intérêt est fixé audit matériau isolant.
